(19) Europäisches Patentamt
European Patent Office
Office européen des brevets

(11) EP 0 860 213 A2

(12) EUROPÄISCHE PATENTANMELDUNG

(43) Veröffentlichungstag:
**26.08.1998 Patentblatt 1998/35**

(51) Int. Cl.[6]: **B05D 5/00**, A61L 29/00, A61L 27/00

(21) Anmeldenummer: **97121482.0**

(22) Anmeldetag: **05.12.1997**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **03.01.1997 DE 19700081**
**03.01.1997 DE 19700082**
**03.01.1997 DE 19700083**
**29.07.1997 DE 19732588**

(71) Anmelder:
**HÜLS AKTIENGESELLSCHAFT**
**45764 Marl (DE)**

(72) Erfinder:
- **Ottersbach, Peter, Dr.**
  **57570 Windeck (DE)**
- **Helary, Gerard, Dr.**
  **95640 Santeuil (FR)**
- **Jozefowicz, Marcel, Prof**
  **60260 Lamorlaye (FR)**
- **Migonney, Veronique, Dr.**
  **95600 Eaubonne (FR)**
- **Vairon, Jean-Pierre, Prof.**
  **92340 Bonzy la Reine (FR)**

## (54) Bioaktive Beschichtung von Oberflächen

(57) Die Erfindung betrifft ein Verfahren zur Herstellung von bioaktiven, kovalent fixierten Beschichtungen auf der Oberfläche von Substraten, bei dem man ein Beschichtungspolymer, das

(i) mindestens ein Monomer der allgemeinen Formel $R\text{-}(A)_a$ (I), in der

R einen ein- oder zweifach olefinisch ungesättigten organischen Rest, vorzugsweise einen Kohlenwasserstoffrest, mit der Wertigkeit a bedeutet,
A eine Carboxylgruppe -COOH, Schwefelsäuregruppe $-OSO_2OH$, Sulfonsäuregruppe $-SO_3H$, Phosphorsäuregruppe $-OPO(OH)_2$, Phosphonsäuregruppe $-PO(OH)_2$, Phosphorigsäuregruppe $-0P(OH)_2$, phenolische Hydroxylgruppe oder ein Salz einer der genannten sauren Gruppen bezeichnet, und
a für 1, 2 oder 3 steht; und

(ii) mindestens ein Monomer mit einer UV-strahlungssensitiven Gruppe
einpolymerisiert enthält, strahleninduziert auf eine gegebenenfalls aktivierte Substratoberfläche pfropft. Auf diese Weise beschichtete Gegenstände finden Verwendung im medizinischen oder biotechnischen Bereich.



Printed by Xerox (UK) Business Services
2.16.3/3.4

## Beschreibung

Die Erfindung betrifft ein Verfahren zur Beschichtung von Oberflächen, vorzugsweise von Polymersubstraten, mit Beschichtungspolymeren, die aufgrund der Anwesenheit bestimmter funktioneller Gruppen bioaktiv und kovalent, also dauerhaft auf der Oberfläche fixiert sind. Die Beschichtungen sind in jedem Fall bakterienabweisend und können zudem zellproliferationsinhibierend oder zellproliferationsfördernd eingestellt werden. Die Erfindung betrifft weiterhin Erzeugnisse mit derart beschichteten Oberflächen u.a. für medizinische oder biotechnische Zwecke.

Die Ansiedelung und Vermehrung von Bakterien auf Oberflächen ist eine in der Regel unerwünschte Erscheinung, die häufig mit nachteiligen Folgen verbunden ist. So können in der Trinkwasser- und Getränketechnik Bakterienpopulationen zu einer gesundheitsgefährdenden Qualitätsminderung führen. Bakterien auf oder in Verpackungen bewirken häufig den Verderb von Lebensmitteln oder verursachen sogar Infektionen bei dem Verbraucher. In steril zu betreibenden biotechnischen Anlagen stellen systemfremde Bakterien ein erhebliches prozeßtechnisches Risiko dar. Solche Bakterien können mit Rohstoffen eingetragen werden oder bei mangelhafter Sterilisation in allen Anlageteilen zurückbleiben. Teile der Bakterienpopulation können sich durch Adhäsion dem normalen Flüssigkeitsaustausch beim Spülen und Reinigen entziehen und sich im System vermehren.

Weiterhin sind Bakterienansiedelungen in Wasseraufbereitungsanlagen (z.B. zur Entsalzung durch Membranen) oder auch in Behältern bekannt. die mit gelösten oder flüssigen unverdünnten organischen Substanzen gefüllt sind und für Bakterienpopulationen vorteilhafte Bedingungen aufweisen. Solche mikrobiellen Belegungen können in erheblichem Umfang zur Blockierung und/oder korrosiven Zerstörung der Anlage führen.

Besondere Bedeutung kommt dem Schutz vor Bakterienanhaftung und -ausbreitung in der Ernährung, der Pflege, hier insbesondere in der Altenpflege, und in der Medizin zu. Bei Massenbeköstigungen oder -ausschank existieren besonders dann erhebliche Risiken, wenn zur Vermeidung von Abfall von Einweggeschirr abgesehen wird und eine nur unzureichende Reinigung des Mehrweggeschirrs erfolgt. Die schädliche Ausbreitung von Bakterien in lebensmittelführenden Schläuchen und Rohren ist ebenso bekannt wie die Vermehrung in Lagerbehältern sowie in Textilien in feuchter und warmer Umgebung, z.B. in Bädern. Solche Einrichtungen sind bevorzugte Lebensräume für Bakterien, ebenso wie bestimmte Oberflächen in Bereichen mit hohem Publikumsverkehr, so z.B. in öffentlichen Verkehrsmitteln, Krankenhäusern, Telefonzellen, Schulen und insbesondere in öffentlichen Toiletten.

In der Alten- und Krankenpflege erfordern die häufig geminderten Abwehrkräfte der Betroffenen sorgfältige Maßnahmen gegen Infektionen, insbesondere auf Intensivstationen und in der häuslichen Pflege.

Besondere Sorgfalt bedarf die Verwendung medizinischer Gegenstände und Geräte bei medizinischen Untersuchungen. Behandlungen und Eingriffen, vor allem dann, wenn derartige Geräte oder Gegerstände mit lebendem Gewebe oder mit Körperflüssigkeiten in Kontakt kommen. Im Falle von Langzeit- oder Dauerkontakten, beispielsweise bei Implantaten, Kathetern, Stents, Herzklappen und Herzschrittmachern, können Bakterienkontaminationen zu einem lebensbedrohenden Risiko für den Patienten werden.

Es wurde bereits auf vielfältige Weise versucht, die Ansiedelung und Ausbreitung von Bakterien auf Oberflächen zu unterbinden. In J.Microbiol.Chemoth. 31 (1993), 261-271, beschreiben S.E.Tebbs und T.S.J.Elliot lackartige Beschichtungen mit quaternären Ammoniumsalzen als antimikrobiell wirkenden Komponenten. Es ist bekannt, daß diese Salze von Wasser, wäßrigen oder anderen polaren Medien sowie von Körperflüssigkeiten aus dem Beschichtungsmaterial herausgelöst werden und ihre Wirkung somit nur von kurzer Dauer ist. Dies gilt gleichermaßen für die Einarbeitung von Silbersalzen in Beschichtungen, so beschrieben in WO 92/18098.

T. Ouchi und Y. Ohya beschreiben in Progr.Polym.Sci. 20 (1995), 211 ff., die Immobilisierung von bakteriziden Wirkstoffen auf Polymeroberflächen durch kovalente Bindung oder ionische Wechselwirkungen. Häufig sind in solchen Fällen die keimtötenden Wirkungen gegenüber dem reinen Wirkstoff deutlich reduziert. Heteropolare Bindungen erweisen sich oft als nicht hinreichend stabil. Darüber hinaus führt die Keimabtötung in der Regel zu unerwünschten Ablagerungen auf den Oberflächen, die die weitere bakterizide Wirkung maskieren und die Grundlage für eine nachfolgende Bakterienbesiedelung bilden.

W. Kohnen et al. berichten in ZBl. Bakt. Suppl. 26, Gustav Fischer Verlag, Stuttgart-Jena-New York, 1994, Seiten 408 bis 410, daß die Adhäsion von <u>Streptococcus epidermidis</u> auf einem Polyurethanfilm vermindert wird, wenn der Film durch eine Glimmentladung in Gegenwart von Sauerstoff vorbehandelt und dann mit Acrylsäure gepfropft wird.

Bei vielen medizinischen Anwendungen kommt es nicht nur darauf an, daß Oberflächen bakterienfrei gehalten werden, vielmehr spielt auch die Besiedlung mit Zellen eine Rolle. In der modernen Medizin werden häufig körperfremde Gegenstände so eingestzt, daß sie mittel- oder langfristig mit Gewebe oder Körperflüssigkeiten in Berührung kommen. Als Beispiele seien Implantate, wie Herzschrittmacher, Stents und Prothesen, sowie Nahtmaterialien, Drainageschläuche und Katheter genannt. Solche Gegenstände können u.a. aus Metallen, Keramik und/oder Polymeren bestehen. Diese Materialien müssen biokompatibel, d.h. verträglich mit dem Gewebe und/ oder den Gewebeflüssigkeiten sein, mit denen sie in Kontakt sind. Es sind zahlreiche Verfahren bekannt geworden, die Polymere biokompatibel machen oder die Biokompatibilität verbessern sollen. Eine dieser Methoden ist die Besiedelung der Polymeroberflächen mit menschlichen Zellen.

Auf der anderen Seite gibt es auch medizinische Anwendungen, bei denen eine Besiedelung der Oberfläche solcher körperfremder Gegenstände mit menschlichen Zellen ausgesprochen unerwünscht ist. So sind Zellbesiedelungen bei mittelfristig intrakorporal applizierten Kathetern ebenso schädlich wie bei langfristig implantierten Stents oder Herzklappen. In WO 94/16648 wird ein Verfahren beschrieben, durch das die Haftung und Proliferation von Zellen auf der Oberfläche von implantierten Augenlinsen aus Polyermaterial verhindert werden soll. Gemäß EP 0 431 213 werden Polymere mit zellabweisenden Eigenschaften ausgestattet, indem ihre Oberfläche mit starken Mineralsäuren hydrophiliert wird. Dies führt zu einer Verringerung der Zelladhäsion.

Die nachträgliche chemische Modifikation von Polymeroberflächen ist jedoch meist nicht gleichmäßig. Es bleiben häufig nicht oder nicht ausreichend behandelte Stellen zurück. die Ausgangspunkte für eine Zellbesiedelung bilden. Weiterhin sind die zellabweisenden Eigenschaften der behandelten Oberflächen häufig nicht nachhaltig.

Der vorliegenden Erfindung lag die Aufgabe zugrunde, ein verbessertes Verfahren zur bioaktiven Beschichtung von Oberflächen zu entwickeln, mit dem Oberflächen auf physiologish verträgliche Weise von Bakterien, z.B. von Kokken, nachhaltig weitgehend frei gehalten werden, ohne daß die mechanischen Eigenschaften der behandelten Materialien dadurch verändert werden oder andere der geschilderten Nachteile des Standes der Technik eintreten. Gemäß einer weiteren Aufgabe der Erfindung sollte ein Verfahren gefunden werden, nach dem die bakterienabweisenden Beschichtungen zugleich wahlweise zellproliferationsinhibierend oder zellproliferationsfördernd eingestellt werden können.

Es wurde überraschenderweise gefunden, daß sich bakterienabweisende, kovalent fixierte Beschichtungen auf der Oberfläche von Substraten, insbesondere von Polymersubstraten, vorteilhaft herstellen lassen, wenn man ein Beschichtungspolymer, das

(i) mindestens ein Monomer der allgemeinen Formel

$$R\text{-}(A)_a\ , \qquad \text{Formel I}$$

in der

R einen ein- oder zweifach olefinisch ungesättigten organischen Rest mit der Wertigkeit a bedeutet,
A eine Carboxylgruppe -COOH, Schwefelsäuregruppe $-OSO_2OH$, Sulfonsäuregruppe $-SO_3H$, Phosphorsäuregruppe $-OPO(OH)_2$, Phosphonsäuregruppe $-PO(OH)_2$, Phosphorigsäuregruppe $-0P(OH)_2$, phenolische Hydroxylgruppe oder ein Salz einer der genannten Gruppen bezeichnet, und
a für 1, 2 oder 3 steht; und

(ii) mindestens ein UV-strahlungssensitives Monomer einpolymerisiert enthält,
strahleninduziert auf eine gegebenenfalls aktivierte Substratoberfläche aufpfropft.

Der organische Rest R kann Kohlenwasserstoffstruktur besitzen oder außer Kohlenstoff und Wasserstoff weitere Atome enthalten, z.B. Sauerstoff-, Stickstoff- und/oder Siliciumatome.

Wenn das Beschichtungspolymer ein Monomer I mit einer Carboxylgruppe oder einem Salz der Carboxylgruppe (also einer Carboxylatgruppe) enthält, weist entweder dieses Monomer zweckmäßig mindestens einen weiteren Rest A mit einer anderen der für A genannten Bedeutungen auf oder enthält das Beschichtungspolymer zweckmäßig mindestens ein weiteres Monomer I, in dem A eine andere der für A genannten Bedeutungen hat. Auf diese Weise wird die relativ schwach ausgeprägte bakterienabweisende Wirkung der Carboxylgruppe bzw. ihrer Salze verstärkt.

Unter den Salzen der für A genannten Gruppen werden die Alkalisalze und insbesondere die Natriumsalze bevorzugt.

Das gemeinsame Kennzeichen der Monomeren der Formel I ist, daß sie 1 oder 2 olefinische Doppelbindungen sowie mindestens eine saure Gruppe oder ein Salz einer sauren Gruppe aufweisen.

Durch Plasma-induzierte Pfropfpolymerisation erzeugte Beschichtungen auf verschiedenen Substraten sind z.B. aus B.Lassen et al., Clinical Materials 11 (1992), 99 bis 103, bekannt und auf Biokompatibilität untersucht worden. Dabei wurden aber lediglich UV-strahlungssensitive Monomere gepfropft, und eine Pfropfung auf aktivierte Substratoberflächen wird nicht erwähnt. Zudem ist Plasma kein optimaler Polymerisationsinitiator. H.Yasuda spricht dementsprechend in J.Polym.Sci.: Macromolecular Review, Vol. 16 (1981), 199-293, von der undefinierten und nicht kontrollierbaren Chemie der Plasma-Polymerisation. Dies mag für manche Zwecke akzeptabel sein, ist jedoch für medizinische und biotechnische Anwendungen problematisch, weil es gerade hier auf reproduzierbare Beschichtungen von gleichbleibend hoher Qualität besonders ankommt.

Die erfindungsgemäß modifizierten Oberflächen vermindern die Adhäsion von Bakterien in einem hohen Maße auch über längere Zeit. Zu den Bakterienstämmen, deren Adhäsion gemäß der Erfindung vermindert oder verhindert wird, zählen Staphylococcus aureus, Staphylococcus epidermidis, Streptococcus pyogenes, Klebsiella pneumoniae,

Pseudomonas aeringinosa, Escherichia coli und Enterobacter faecium. Die beschichteten Oberflächen sind frei von migrationsfähigen und/oder extrahierbaren Monomer- und Oligomeranteilen. Unerwünschte Nebenwirkungen durch freigesetzte körperfremde Stoffe oder durch abgetötete Bakterien werden von vornherein vermieden. Die Oberflächen der aufgepfropften Beschichtungen weisen somit eine hervorragende physiologische Verträglichkeit auf. Die besonderen Bedingungen, unter denen die Oberflächen zusätzlich zu ihren bakterienhemmenden Eigenschaften zellproliferationsinhibierend oder zellproliferationsfördernd wirken, werden später erläutert.

Bei dem erfindungsgemäßen Verfahren werden die gegebenenfalls aktivierten Substratoberflächen zunächst mit dem Beschichtungspolymeren beschichtet, und die Beschichtung wird anschließend unter Einwirkung von UV-Licht durch schonende Pfropfung des vorgebildeten Beschichtungspolvmeren auf der Substratoberfläche kovalent, also dauerhaft fixiert.

1. Die Beschichtungspolymeren

Die Beschichtungspolymeren weisen mindestens ein einpolymerisiertes Monomer der allgemeinen Formel I auf, dessen funktionelle Gruppe A für die bioaktiven Eigenschaften der polymeren Beschichtung verantwortlich ist. Zu diesen Monomeren I zählen Monomere der allgemeinen Formeln II und III,

$$(C_nH_{2n-q-x})(COOR^1)_x \qquad \text{Formel II}$$

$$(C_nH_{2n-q-x})(SO_3R^1)_x, \qquad \text{Formel III}$$

die bevorzugte Monomere für die Herstellung der Beschichtungspolymeren sind. Beschichtungspolymere, die sowohl mindesters ein Monomer II als auch mindestens ein Monomer III enthalten, sind besonders stark bakterienabweisend. wobei die Reste $(C_nH_{2n-q-x})$ gleich oder verschieden sein können. In den Formeln II und III stehen

<u>n</u> jeweils unabhängig für eine ganze Zahl von 2 bis einschließlich 6;
<u>x</u> jeweils unabhängig für 1 oder 2;
<u>q</u> jeweils unabhängig für 0 oder 2; und
bedeutet der Rest $R^1$ jeweils unabhängig -H oder ein Äquivalent eines Metallions, vorteilhaft ein Alkalimetallion und insbesondere ein Natriumion.

Den gegebenen Definitionen entsprechend bedeutet der Rest $(C_nH_{2n-q-x})$- jeweils unabhängig einen geradkettigen oder verzweigten einwertigen Alkenylrest (q=0, x=1) oder Alkadienylrest (q=2, x=1) oder einen zweiwertigen Alkenylenrest (q=0, x=2) oder Alkadienylenrest (q=2, x=2).

Anstelle von zwei Monomeren II und III kann man auch nur ein Monomer (II + III) einsetzen, das die Gruppen $COOR^1$ und $SO_3R^1$ in demselben Molekül enthält.

Auch vom Benzol abgeleitete Monomerkomponenten der allgemeinen Formel IV

$$(C_6H_{6-b-c-d})B_bR^3{}_c(OH)_d \qquad \text{Formel IV}$$

fallen unter die Formel I und können vorteilhaft als Monomere in dem Beschichtungspolymer enthalten sein, wobei

B jeweils unabhängig einen ein- oder zweifach ungesättigten geradkettigen oder verzweigten Rest der Formeln $-(C_nH_{2n-1-q-x})(COOR^1)_x$ oder $-(C_nH_{2n-1-q-x})(SO_3R^1)_x$ bedeutet, wobei $R^1$, <u>n</u>, <u>q</u> und <u>x</u> wie zuvor definiert sind;
$R^3$ jeweils unabhängig $C_{1-4}$-Alkyl, $-NH_2$, -COOH, $-SO_3H$, $-OSO_3H$, $-OPO(OH)_2$, $-PO(OH)_2$, $-OP(OH)_2$, $-OPO(O^-)OCH_2CH_2N^+(CH_3)_3$, $-PO(O^-)OCH_2CH_2N^+(CH_3)_3$, $-OP(O^-)OCH_2CH_2N^+(CH_3)_3$ oder ein Salz, zweckmäßig ein Alkalisalz und insbesondere ein Natriumsalz bedeutet;
<u>b</u> für 1, 2 oder 3 steht;
<u>c</u> für 0, 1, 2 oder 3 steht; und
<u>d</u> für 0, 1, 2 oder 3 steht;
mit der Maßgabe, daß $\bar{b} + \bar{c} + \bar{d} \leq 6$ , vorteilhaft $\leq 4$ ist.

Andere geeignete Monomere für die Herstellung der Beschichtungspolymeren, die auf die aktivierte Substratoberfläche gepfropft werden, sind der Formel I entsprechende olefinisch ungesättigte saure Schwefelsäureester und deren Salze; Sulfonsäuren und deren Salze; Phosphonsäuren und deren neutrale oder saure Salze; Phosphorsäureester und deren neutrale oder saure Salze; und Phosphorigsäureester sowie deren neutrale oder saure Salze. Schließlich seien noch der Formel I entsprechende 1 bis 3-wertige (oder -basische) Phenole mit olefinischen Gruppen sowie deren Salze als geeignete Monomere erwähnt.

Natürlich können die Beschichtungspolymeren in jedem Fall und nicht nur wie im zuvor erwähnten Fall der Monomeren II und III verschiedene Reste A enthalten, was man durch entsprechende Auswahl der Monomeren erreicht.

Von den für die Herstellung der Beschichtungspolymeren geeigneten Monomeren der allgemeinen Formeln I bis IV, die einen oder mehrere gleiche oder verschiedene Reste A im Molekül enthalten, seien beispielsweise genannt: Acrylsäure, Methacrylsäure, 4-Vinylsalicylsäure, Itaconsäure, Vinylessigsäure, Zimtsäure, 4-Vinylbenzoesäure, 2-Vinylbenzoesäure, Sorbinsäure, Kaffeesäure, Maleinsäure, Methylmaleinsäure, Dimethylmaleinsäure, Dihydroxymaleinsäure, Isocrotonsäure, Fumarsäure, Methylfumarsäure, Allylessigsäure, 4-Vinylsalicylsäure sowie die Alkalisalze, insbesondere die Natriumsalze der genannten Säuren; Vinylsulfonsäure, Allylsulfonsäure, Methallylsulfonsäure, Vinylsulfonsäure, 4-Styrolsulfonsäure, 2-Styrolsulfonsäure, Vinyltoluolsulfonsäure, 4-Carboxylstyrolsulfonsäure sowie die Alkalisalze und insbesondere die Natriumsalze der genannten Sulfonsäuren; diprimäres Butadien-(1,3)-diol-(1,4)-diphosphat, 4- und 2-Vinylphenol, 2-Allylhydrochinon und 4-Vinylresorcin sowie die entsprechenden Salze.

Neben den Monomeren der allgemeinen Formeln I bis IV können auch weitere Monomere in dem Beschichtungspolymer enthalten sein, die nicht oder nicht nennenswert zu den bioaktiven Eigenschaften der Beschichtung beitragen. Dazu gehören z.B. Vinylether, wie Vinylmethylether und Vinylbutylether; Vinylester, wie Vinylacetat und Vinylpropionat; Vinylketone, wie Vinylethylketon und Vinyl-n-butylketon; Nitrile, wie Acrylnitril und Methacrylnitril; Carbonamide, wie Acrylamid, N,N-Dimethylacrylamid und Methacrylamid; Carbonsäureanhydride, wie Maleinsäureanhydrid; Ester, wie Methylacrylat, Ethylacrylat, 2-Ethylhexylacrylat, Methylmethacrylat, 2-Hydroxyethylacrylat, 2-(2'-Hydroxyethoxy)ethylacrylat, 2-Hydroxy-1-methylethylacrylat, 2-N,N-Dimethylaminoethylacrylat, n-Propylmethacrylat, 2-Hydroxyethylmethacrylat, 2-(2'-Hydroxyethoxy)ethylmethacrylat, 2-Hydroxy-1-methylethylmethacrylat, 2-N,N-Dimethylaminoethylmethacrylat, Diethylenglykolmethacrylat, Triethylenglykoldiacrylat, Ethylvinylsulfonat und Vinylsulfonsäure-2-hydroxyethylester; Olefine und Diolefine, wie 1-Buten, 1-Hexen, 1-Octen, 1,3-Butadien, Isopren und Chloropren; Vinylsiloxane und andere siliciumhaltige Vinylmonomere, wie Tris(trimethylsiloxy)methacryloylpropylsilan und Tris(trimethylsiloxy)acryloylpropylsilan. Diese oder andere Monomere können sogar in überwiegender Menge vorhanden sein, z.B. bis zu 90 Mol-% ausmachen.

Monomere mit Gruppen, die in Gruppen A überführbar sind, können als potentielle Monomere der Formel I angesehen werden. Dazu gehören in erster Linie Ester, Anhydride, Säureamide und Nitrile, die zumindest an der Oberfläche - und nur auf diese kommt es hinsichtlich der bioaktiven Eigenschaften an - in bekannter Weise sauer oder alkalisch zu Carboxyl- oder Carboxylatgruppen bzw. Sulfonsäure- oder Sulfonatgruppen verseift werden können. Solange dies nicht geschehen ist, gelten diese Monomere als weitere Monomere im Sinne dieser Beschreibung.

Bevorzugte Beschichtungspolymere enthalten einpolymerisiert (a) Monomere mit Carbonsäure- und/oder Carboxylatgruppen sowie (b) Monomere mit Sulfonsäure- und/oder Sulfonatgruppen, wobei die molaren Anteile dieser Monomeren in den Beschichtungspolymeren zusammen in der Regel 5 bis 40 %, vorteilhaft 5 bis 30 % und insbesondere 15 bis 20 % betragen. Das Molverhältnis der Monomeren (a) zu den Monomeren (b) beträgt vorteilhaft <10, insbesondere <5. Ausgeprägte bakterienabweisende Eigenschaften zeigen Beschichtungspolymere, in denen das genannte Verhältnis 0,5 bis 10, vorteilhaft 0,5 bis 5 beträgt. Wenn das Verhältnis im Bereich von 0,4 bis 3, vorteilhaft von 0,4 bis 2 liegt, zeigen die Beschichtungspolymeren neben der bakterienabweisenden Wirkung starke zellproliferationsinhibierende Eigenschaften. Liegt das Verhältnis im Bereich von 2 bis 10, vorteilhaft von > 3 bis 5, weisen die Beschichtungspolymeren überraschenderweise zellproliferationsfördernde Eigenschaften auf, Zellproliferationsinhibierend im Sinne der Erfindung ist eine Beschichtung dann, wenn die Haftung und Vermehrung von Säugetierzellen auf der Beschichtung gegenüber dem unbeschichteten Substrat vermindert ist. Als zellproliferationsfördernd im Sinne der Erfindung gilt eine Beschichtung dann, wenn die Haftung und Vermehrung von Säugetierzellen auf der Beschichtung im Vergleich zu dem unbeschichteten Substrat verbessert oder jedenfalls weniger stark beeinträchtigt ist als die Haftung von Bakterien.

Unter dem Gesichtspunkt der Verträglichkeit gibt es drei mögliche Zweierkombinationen aus den genannten Gruppen, nämlich Carboxyl- und Sulfonsäuregruppen, Carboxyl- und Sulfonatgruppen sowie Carboxylat- und Sulfonatgruppen, weiterhin zwei mögliche Dreierkombinationen, nämlich Carboxyl-, Carboxylat- und Sulfonatgruppen sowie Carboxyl-, Sulfosäure- und Sulfonatgruppen. Alle diese Kombinationen charakterisieren vorteilhafte Beschichtungen im Sinne der Erfindung. Natürlich ist es auch möglich, wie zuvor erwähnt, im Beschichtungspolymer vorliegende Gruppen nachträglich in funktionelle Gruppen A umzuwandeln, z.B. Carbonamid-Gruppen (z.B. von Acrylamid stammend) durch Hydrolyse in saurem Medium in Carboxylgruppen. Weiterhin kann man Carboxylgruppen und Sulfonsäuregruppen durch Neutralisieren (z.B. in Phosphatpuffern) in Carboxylat- bzw. Sulfonatgruppen überführen. In jedem Fall ändert sich dadurch das erwähnte molare Verhältnis der Monomeren (a) und (b), möglicherweise mit quantitativen oder auch qualitativen Folgen für die Eigenschaften des Beschichtungspolymers.

Ein wesentlicher Bestandteil der Beschichtungspolymeren ist ein einpolymerisiertes Monomer mit UV-strahlungssensitiver Gruppe. Als solche eignen sich alle Monomeren, die nach dem Einpolymerisieren noch mindestens eine reaktiottsfähige Doppelbindung aufweisen, die die Pfropfung des Beschichtungspolymers auf die aktivierte Substratoberfläche ermöglicht. Als Beispiele seien vinylische Cinnamoyl- oder Furylderivate und insbesondere Cinnamoylethylacrylat oder -methacrylat genannt. Das UV-strahlungssensitive Monomer wird vorteilhaft in Mengen von 1 bis 20

Mol-%, vorteilhaft von 3 bis 15 Mol-%, bezogen auf die gesamten Monomeren, eingesetzt. Bei der radikalisch initiierten Polymerisation bleibt die zum Benzolring α-ständige Doppelbindung als UV-strahlungssensitive Gruppe für die spätere Pfropfung erhalten.

Die Polymeren werden in üblicher Weise durch radikalisch initiierte Polymerisation hergestellt. vorteilhaft durch Lösungs- oder Emulsionspolymerisation. Geeignete Lösemittel sind z.B. Wasser; Ketone, wie Aceton, Methylethylketon und Cyclohexanon; Ether, wie Diethylether, Tetrahydrofuran und Dioxan; Alkohole, wie Methanol, Ethanol, n- und iso-Propanol, n- und iso-Butanol sowie Cyclohexanol; stark polare Lösemittel, wie Dimethylformamid, Dimethylacetamid und Dimethylsulfoxid; Kohlenwasserstoffe, wie Heptan, Cyclohexan, Benzol und Toluol; Halogenkohlenwasserstoffe, wie Dichlormethan und Trichlormethan; Ester, wie Ethylacetat, Propylacetat und Amylacetat; sowie Nitrile, wie Acetonitril.

Geeignete Polymerisationsinitiatoren sind z.B. Azonitrile, Alkylperoxide, Acylperoxide, Hydroperoxide, Peroxyketone, Peroxyester und Percarbonate sowie alle üblichen Photoinitiatoren. De Polymerisation wird thermisch, z.B. durch Erhitzen auf 60 bis 100°C, oder durch Strahlung mit entsprechender Wellenlänge eingeleitet. Nach Beendigung der exothermen Polymerisationsreaktion wird das Polymer in üblicher Weise vom Lösemittel abgetrennt, beispielsweise durch Fällung mittels Wasser, sofern das Lösemittel wasserlöslich ist. Durch Extraktion mit einem geeigneten Lösemittel können monomere oder oligomere Bestandteile entfernt werden.

2. Die Substratmaterialien

Als Substratmaterialien eignen sich vor allem polymere Substrate, wie Polyurethane, Polyamide, Polyester und -ether, Polyetherblockamide, Polystyrol, Polyvinylchlorid, Polycarbonate, Polyorganosiloxane, Polyolefine, Polysulfone, Polyisopren, Polychloropren, Polytetrafluorethylen (PTFE), Polysiloxane, entsprechende Copolymere und Blends sowie natürliche und synthetische Kautschuke, mit oder ohne strahlungssensitive Gruppen. Das erfindungsgemäße Verfahren läßt sich auch auf Oberflächen von lackierten oder anderweitig mit Polymeren beschichteten Metall-, Glas- oder Holzkörpern anwenden. Die Oberflächen der Substratmaterialien werden zweckmäßig vor der Beschichtung mit den Beschichtungspolymeren in bekannter Weise mit einem Lösemittel von anhaftenden Ölen, Fetten und anderen Verunreinigungen befreit. Sie können, müssen aber nicht vor der Beschichtung aktiviert werden, wie in der Folge beschrieben. Die Aktivierung führt in manchen Fällen zu einer besseren Haftung der gepfropften Beschichtung auf dem Substratmaterial. In der Regel unterscheiden sich aber die Beschichtungen auf nicht aktivierten Substratoberflächen hinsichtlich der biologischen Wirkungen und in bezug auf die Haftung praktisch nicht von den Beschichtungen auf aktivierten Oberflächen.

3. Die Aktivierung der Substratoberflächen

Von den Methoden, nach denen die Oberflächen der polymeren Substrate gegebenenfalls aktiviert werden, seien die folgenden genannt

3.1 Bei der Herstellung der Substratpolymeren können Monomere mit UV-strahlungssensitiven Gruppen einpolymerisiert werden. ähnlich wie für die Beschichtungspolymeren beschrieben. Hierfür kommen dieselben Monomeren in Betracht, die auch in den Beschichtungspolymeren enthalten sein können. Diese Monomeren können z.B. in Mengen von 1 bis 20 Mol-%, vorteilhaft von 3 bis 15 Mol-% angewandt werden. Solche strahlungssensitiv modifizierten Polymere können in üblicher Weise durch radikalisch initiierte Polymerisation in Lösung. Emulsion oder Suspension hergestellt werden.

3.2 Alternativ kann die Aktivierung von Standardpolymeren ohne UV-strahlungssensitive Gruppen durch UV-Strahlung, z.B. im Wellenlängenbereich von 100 bis 400 nm, vorzugsweise von 125 bis 310 nm erfolgen. Eine geeignete Strahlenquelle ist z.B. ein UV-Excimer-Gerät HERAEUS Noblelight, Hanau, Deutschland. Aber auch Quecksilberdampflampen eignen sich zur Substrataktivierung, sofern sie erhebliche Strahlungsanteile in den genannten Bereichen emittieren. Die Expositionszeit beträgt, je nach Strahlungsintensität und Wellenlänge, im allgemeinen 0,1 Sekunden bis 20 Minuten, vorzugsweise 1 Sekunde bis 10 Minuten. Es hat sich gezeigt, daß die Anwesenheit von Sauerstoff vorteilhaft ist. Die bevorzugten Sauerstoffdrücke liegen zwischen $2x10^{-5}$ und $2x10^{-2}$ bar. Man arbeitet beispielsweise in einem Vakuum von $10^{-4}$ bis $10^{-1}$ bar oder unter Verwendung eines Inertgases, wie Helium, Stickstoff oder Argon, mit einem Sauerstoffgehalt von 0,02 bis 20 Promille.

3.3 Die Aktivierung kann erfindungsgemäß auch durch ein Hochfrequenz- oder Mikrowellenplasma (Hexagon, Fa. Technics Plasma, 85551 Kirchheim, Deutschland) in Luft, Stickstoff- oder Argon-Atmosphäre erreicht werden. Die Expositionszeiten betragen im allgemeinen 30 Sekunden bis 30 Minuten. vorzugsweise 2 bis 10 Minuten. Der Energieeintrag liegt bei Laborgeräten zwischen 100 und 500W, vorzugsweise zwischen 200 und 300W.

3.4 Weiterhin lassen sich auch Korona-Geräte (Fa. SOFTAL, Hamburg. Deutschland) zur Aktivierung verwenden. Die Expositionszeiten betragen in diesem Falle in der Regel 1 Sekunde bis 10 Minuten, vorzugsweise 1 bis 60 Sekunden.

3.5 Die Aktivierung durch Elektronen- oder gamma-Strahlen (z.B. aus einer Kobalt-60-Quelle) ermöglicht kurze Expositionszeiten, die im allgemeinen 0,1 bis 60 Sekunden betragen.

3.6 Beflammungen von Oberflächen führen ebenfalls zu deren Aktivierung. Geeignete Geräte, insbesondere solche mit einer Barriere-Flammenfront, lassen sich auf einfache Weise bauen oder beispielsweise beziehen von der Fa. ARCOTEC, 71297 Mönsheim, Deutschland. Sie können mit Kohlenwasserstoffen oder Wasserstoff als Brenngas betrieben werden. In jedem Fall muß eine schädliche Überhitzung des Substrats vermieden werden, was durch innigen Kontakt mit einer gekühlten Metallfläche auf der von der Beflammungsseite abgewandten Substratoberfläche leicht erreicht wird. Die Aktivierung durch Beflammung ist dementsprechend auf verhältnismäßig dünne, flächige Substrate, wie Folien, beschränkt. Die Expositionszeiten belaufen sich im allgemeinen auf 0,1 Sekunde bis 1 Minute, vorzugsweise 0,5 bis 2 Sekunden, wobei es sich ausnahmslos um nicht leuchtend Flammen handelt und die Abstände der Substratoberflächen zur außeren Flammenfront 0,2 bis 5 cm, vorzugsweise 0,5 bis 2 cm betragen.

3.7 Weiterhin lassen sich die Substratoberflächen auch durch Behandlung mit starken Säuren oder starken Basen aktivieren. Von den geeigneten starken Säuren seien Schwefelsäure, Salpetersäure und Salzsäure genannt. Man kann z.B. Polyamide 5 Sekunden bis 1 Minute mit konzentrierter Schwefelsäure bei Raumtemperatur behandeln. Als starke Basen eignen sich besonders Alkalimetallhydroxide in Wasser oder einem organischen Lösemittel. So kann man z.B. verdünnte Natronlauge 1 bis 60 Minuten bei 20 bis 80°C auf die Substratoberfläche einwirken lassen. Alternativ können beispielsweise Polyamide aktiviert werden, indem man 2 %-iges KOH in Tetrahydrofuran 1 Minute bis 30 Minuten auf die Oberfläche einwirken läßt.

3.8 In manchen Fällen, z.B. bei hochhydrophoben Polymeren, kann es empfehlenswert sein, die Substratoberflächen durch eine Kombination aus zwei oder mehr der genannten Methoden zu aktivieren. Ganz allgemein hat sich eine Substrataktivierung bewährt, bei der der Einbau UV-strahlungssensitiver Gruppen (3.1) mit einer UV-Bestrahlung (3,2) kombiniert wird.

4. Die Beschichtung durch Pfropfpolymerisation

Nach einer der unter 3,2 bis 3,8 beschriebenen aktivierenden Vorbehandlungen werden die Substrate mit den aktivierten Oberflächen zweckmäßig 1 bis 20 Minuten, vorzugsweise 1 bis 5 Minuten der Einwirkung Sauerstoff, z.B. in Form von Luft, ausgesetzt. Alternativ kann man auf die aktivierten Oberflächen auch ähnlich lange ein Lösemittel, wie Tetrahydrofuran, einwirken lassen.

Anschließend werden die (gegebenenfalls auch gemäß 3.1) aktivierten oder aber nicht aktivierten Oberflächen nach bekannten Methoden, wie Tauchen, Sprühen oder Streichen, mit einer Lösung des erfindungsgemäß zu verwendenden Beschichtungspolymeren beschichtet. Als Lösemittel haben sich z.B. Ether, wie Tetrahydrofuran, und/oder stark polare Lösemittel, wie Dimethylsulfoxid, bewährt, doch sind auch andere Lösemittel verwendbar, sofern sie ein ausreichendes Lösevermögen für die Monomeren aufweisen und die Substratoberflächen gut benetzen. Je nach Löslichkeit der Polymeren und gewünschter Schichtdicke der gepfropften Beschichtung beträgt die Konzentrationen des Polymeren in der Lösung im allgemeinen 0,1 bis 50 Gewichtsprozent. Lösungen mit einem Gehalt an Beschichtungspolymer von 3 bis 15 Gew.-%, vorteilhaft von etwa 10 Gew.-% haben sich in der Praxis bewährt und ergeben im allgemeinen in einem Durchgang zusammenhängende, die Substratoberfläche abdeckende Beschichtungen mit Schichtdicken, die mehr als 0,1 μm betragen können.

Nach dem Verdampfen des Lösemittels oder auch schon während des Verdampfens wird die Pfropfung des aufgebrachten Beschichtungspolymeren unter Ausbildung kovalenter Bindungen zur Substratoberfläche zweckmäßig durch Strahlen im kurzwelligen Segment des sichtbaren Bereiches oder im langwelligen Segment des UV-Bereiches der elektromagnetischen Strahlung bewirkt. Gut geeignet ist z.B. die Strahlung eines UV-Excimers der Wellenlängen 250 bis 500 nm, vorzugsweise von 290 bis 320 nm. Auch hier haben sich Quecksilberdampflampen als geeignet erwiesen, sofern sie erhebliche Strahlungsanteile in den angegebenen Bereichen emittieren. Die Expositionszeiten betragen im allgemeinen 10 Sekunden bis 30 Minuten, vorzugsweise 2 bis 15 Minuten.

Bisweilen ist es zweckmäßig, die beschriebenen Arbeitsschritte, gegebenenfalls einschließlich der Aktivierung, zu wiederholen, um mittels einer solchen Mehrschichttechnik eine hermetisch geschlossene und/oder dickere Beschichtung herzustellen. Weiterhin ist es möglich, das gegebenenfalls oberflächenaktivierte Substrat, gegebenenfalls nach der beschriebenen Sauerstoff- oder Lösemittelbehandlung, in die Lösung des erfindungsgemäß zu verwendenden

Beschichtungspolymeren einzutauchen und im getauchten Zustand zu bestrahlen. Durch orientierende Versuche läßt sich unschwer feststellen, bei welchen Bestrahlungszeiten mit einer gegebenen Strahlenquelle und bei welchen, gegebenenfalls längeren Kontaktzeiten von Substrat und Losung die gewünschte Schichtdicke erreicht wird.

Das erfindungsgemäße Verfahren zur bakterienabweisenden Modifizierung der Oberfläche von Substraten und insbesondere von Polymersubstraten gestattet die genaue Einstellung von Molverhältnissen verschiedener funktioneller Gruppen, die zur Inhibierung der Bakterienadhäsion und/oder -ausbreitung sowie zur Regelung des Zellproliferationsverhaltens optimal sind. Es ist ein besonderer Vorteil des Verfahrens und der beschichteten Substrate nach der Erfindung, daß die letzteren zudem eine gute Blutverträglichkeit zeigen. Darüber hinaus bietet das Verfahren den Vorteil, daß bereits bewährte Polymere auf diese Weise unter Beibehaltung ihrer mechanischen Eigenschaften und ihrer Form zusätzlich bakterienabweisend und wahlweise zellproliferationsinhibierend oder zellproliferationsfördernd einstellbar sind. Es sind keine wieiteren Vor- oder Nachbehandlungen erforderlich, sofern eine problemfreie Benetzung und eine chemische Bindung an die Substratoberflächen möglich ist. Hochhydrophobe Kunststoffe bedürfen gegebenenfalls einer hydrophilierenden Vorbehandlung, z.B. durch chemisches Ätzen mit Säuren oder Basen oder durch Plasma-Behandlung, um eine ausreichende Benetzbarkeit durch die Lösung des Beschichtungspolymeren zu erreichen. Die hochhydrophoben Kunststoffe werden dann gleichzeitig hydrophiliert und im Sinne der vorliegenden Erfindung oberflächenaktiviert.

Nach den Verfahren der vorliegenden Erfindung beschichtete und dadurch bakterienabweisend modifizierte Erzeugnisse eignen sich als bioverträgliche Materialien zur Verwendung im biotechnischen oder medizinischen Bereich, z.B. für Lager- oder Verpackungszwecke oder für Schläuche oder Rohrleitungen. Beispiele für medizintechnische Erzeugnisse sind Katheter, Schläuche, Wunddrainagen, Wundverbände, Stents, Intraokularlinsen, Herzschrittmacher und Herzklappen.

Zur wieiteren Erläuterung der vorliegenden Erfindung werden die folgenden Beispiele gegeben, die die Erfindung weiter erläutern, nicht aber ihren Umfang begrenzen sollen, wie er in den Patentansprüchen dargelegt ist. Die in den Beispielen verwendeten Beschichtungspolymeren sind stellvertretend für eine Vielzahl anderer Polymerer mit Monomeren, die unter die Formeln I bis IV fallen.

Beispiele

(1) Untersuchungen zur Bakterienadhäsion

Messung der Bakterienadhäsion an Folien aus Beschichtungspolymeren über Szintillation

Diese Folien sind keine erfindungsgemäßen Produkte, sondern wurden zum Vergleich ihrer bakterienabweisenden Eigenschaften mit denen von bakterienabweisend beschichteten Substratfolien nach der Erfindung hergestellt.

Proben der durch Copolymerisation erhaltenen Beschichtungspolymeren (nachfolgende Beispiele 1 bis 9) werden in einem geeigneten Lösemittel, wie Chloroform, gelöst. Nach Ausgießen in eine Petrischale und Verdampfung des Lösemittels wierden die erhaltenen Polymerfolien über einen Zeitraum von einer Stunde in 1 ml einer Lösung eingetaucht, die aus 0,4 g/l Rinderserumalbumin (BSA), gelöst in phosphatgepufferter physiologischer Kochsalzlösung (PBS), und 20 $\mu$g/ml gereinigtem humanen Fibronektin besteht. Anschließend werden die so mit Fibronektin beschichteten Proben unter kräftigem Rühren 1 Stunde lang bei 37°C in eine Suspension der jeweiligen Bakterien gegeben, die durch Einbau von $^3$H-Thymidin radioaktiv markiert sind. Nach Ablauf der vorgegebenen Zeitspanne werden die überschüssiger Bakterien durch Waschen entfernt, die Polymerfolien 2 mal mit jeweils 3 ml einer PBS-BSA-Lösung abgespült und zur Bestimmung der Anzahl adhärierender Bakterien in ein Schraubglas mit 20 ml Szintillationslösung gelegt. Der Prozentsatz an adhärierenden Bakterien wird über das Verhältnis der in der Probe vorhandenen zu der von den Bakterien ursprünglich eingebrachten Radioaktivität bestimmt. Die Hemmung der Bakterienadhäsion wird prozentual zu der Bakterienadhäsion einer unbehandelten Folie als externem Standard bestimmt.

Messung der Bakterienadhäsion beschichteter Standardfolien über die ATP-Bestimmung (statisch)

Nach einer Adsorption der Bakterienzellen an eingetauchte Polymerfolien werden die nicht adhärierenden Bakterien mit steriler PBS-Pufferlösung weggespült. Aus den adhärierenden Bakterien wird der Zellinhaltsstoff Adenosintriphosphat (ATP) in üblicher Weise extrahiert und mit einer handelsüblichen Testkombination im bioluminometrischen Test bestimmt. Die Anzahl der gemessenen Lichtimpulse ist proportional zur Zahl der adhärierenden Bakterien. Es werden in jedem Fall mehrere Folienstücke eingesetzt. Der mit der unbeschichteten Standardfolie gemessene Wert wird gleich hundert Prozent gesetzt, und die Bakterienadhäsionswerte der bakterienabweisend beschichteten Folien werden als prozentuale Verminderung ausgedrückt.

Messung der Bakterienadhäsion beschichteter Standardfolien über die ATP-Bestimmung (dynamisch)

Die Bakterien werden mit dem zu prüfenden Folienstück in eine Hefeextrakt-Pepton-Glucose-Nährlösung gegeben und 24 Stunden bei 37°C geschüttelt. Im Anschluß daran wird das Folienstück mit Leitungswasser gespült, in einen frischen Kolben mit Nährlösung übertragen und für weitere 24 Stunden bei 37°C geschüttelt. Dieser Zyklus wird noch einmal wiederholt, und das Folienstück wird mit Leitungswasser gespült. Aus den an der Folie adhärierenden Bakterien wird der Zellinhaltsstoff Adenosintriphospat (ATP) extrahiert und mit einer handelsüblichen Testkombination im bioluminometrischen Test bestimmt. Da für die dynamische Messung die gleichen Randbedingungen wie für die statische Messung gültig sind, werden die Bakterienadhäsionswerte der beschichteten Folien als prozentuale Verminderung im Vergleich zu unbeschichteten Standardfolien ausgedrückt.

(2) Untersuchungen zur Zellproliferation

Konditionierung der Polymerfolien (Substratfolien)

Die erfindungsgemäß beschichteten Folien und unbeschichtete Vergleichsfolien werden zwölfmal jeweils 3 Stunden lang bei 37°C in Ethanol gewaschen. Anschließend werden die so vorbehandelten Folien in einer 0,15-molaren Natriumchloridlösung dreimal jeweils 3 Stunden gewaschen und anschließend mit Wasser abgespült. Im folgenden Reinigungsschritt werden die Folien dreimal jeweils 3 Stunden in eine Phosphatpufferlösung gelegt und danach 15 min mit UV-Licht bestrahlt. Die so vorbehandelten Folien werden 16 Stunden bei 37°C in einer DMEM-Lösung (Dulbecco's Modified Eagles Medium) gelagert. Abschließend werden die Folien 16 Stunden in einer mit 0,05 % Antibiotika, 200 mg/l L-Glutamin und 10% eines fötalen Kälberserums versetzten DMEM-Lösung bei 37°C in einer Atmosphäre von 5 % $CO_2$ und 95 % Luft gehalten.

Herstellung der Zellsuspension

Menschliche Fibroblasten (McCoy's) von ATCC Nr. CRL 1996 (Rockville, Maryland, USA) werden in einem DMEM-Medium mit 0.05 % Antibiotika, 200 mg/l L-Glutamin und 10 % eines fötalen Kälberserums bei 37°C in einer Atmosphäre von 5 % $CO_2$ und 95 % Luft gezüchtet. Nach Abtrennen der Zellen vom Nährmedium wird sowohl die Anzahl der lebenden Zellen als auch die Gesamtzahl der Zellen in üblicher Weise bestimmt.

Messung der Zellproliferationseigenschaften

Dann werden die erfindungsgemäß beschichteten Folien und die Vergleichsfolien nach der beschriebenen Vorbehandlung in "Wells" (Vertiefungen in Standardmikrotiterplatten) gegeben und mittels spezieller PTFE-Einsätze, die zuvor mit Ethanol sterilisiert wurden, arretiert. Folien, "Wells" und PTFE-Einsätze werden durch 16 min Bestrahlung mit UV-Licht sterilisiert. Anschließend werden die Polymerfolien mit der Zellsuspension versetzt. Nach einer Inkubation von 8 Tagen bei 37°C werden die Zellen mittels einer Phosphatpufferlösung gereinigt, mit 0,05 Gew.-% Trypsin-EDTA-Lösung abgetrennt, und ihre Anzahl wird optisch oder mit einem Zellcounter ausgezählt.

(3) Herstellung der Beschichtungspolymeren

Beispiel 1

Ein Monomerengemisch aus 65 Mol% Tris(trimethylsiloxy)methacryloyloxypropylsilan (TTMPS), 10 mol% Cinnamoylethylmethacrylat (CEM), 13,7 mol% Methacrylsäure (MA) und 11,3 mol% Dimethyloctylammoniumstyrolsulfonat (DOASS) wird in THF als Lösemittel unter Schutzgas vorgelegt und auf 65°C erhitzt. Nach Erreichen dieser Temperatur werden 0,6 mol% Azobisisobutyronitril zugegeben. Nach einer Reaktionsdauer von 24 Stunden wird das Quaterpolymer durch Entfernen des Lösemittels am Rotationsverdampfer isoliert und anschließend mit Wasser gewaschen. Die NMR-Analyse des Produkts ergibt eine Zusammensetzung von

| TTMPS | CEM | MA | DOASS | |
|---|---|---|---|---|
| 72 | 8,2 | 10,8 | 9 | mol% |

Das Verhältnis von COOH bzw. $COO^-$ zu $SO_3^-$ beträgt 1,2.

Beispiel 2

Ein Monomerengemisch aus 75 Mol% Tris(trimethylsiloxy)methacryloyloxypropylsilan (TTMPS), 10 mol% Cinnamoylethylmethacrylat (CEM), 10 mol% Methacrylsäure (MA) und 5 mol% Dimethyloctylammoniumstyrolsulfonat (DOASS) wird in THF als Lösemittel unter Schutzgas vorgelegt und auf 65°C erhitzt. Nach Erreichen dieser Temperatur werden 0,6 mol% Azobisisobutyronitril zugegeben. Nach einer Reaktionsdauer von 24 Stunden wird das Quaterpolymer durch Entfernen des Lösemittels am Rotationsverdampfer isoliert und anschließend mit Wasser gewaschen. Die NMR-Analyse des Produkts ergibt eine Zusammensetzung von

| TTMPS | CEM | MA | DOASS | |
|---|---|---|---|---|
| 84 | 9,6 | 2,1 | 3,8 | mol% |

Das Verhältnis von COOH bzw. $COO^-$ zu $SO_3^-$ betragt 0,55.

Beispiel 3

55 mol% Methylmethacrylat, 35 mol% Methacrylsäure, 5 mol% Natriumstyrolsulfonat und 5 mol% Cinnamoylethylmethacrylat werden unter Schutzgas in Dimethylsulfoxid gelöst. Nach Erreichen der Reaktionstemperatur von 70°C werden 0,6 mol% Azobisisobutyronitril, gelöst in Dimethylsulfoxid, zugetropft. Nach 18 Stunden Reaktionsdauer wird das Produkt mit Eiswasser ausgefällt und anschließend im Soxhlet mit Aceton und Wasser extrahiert. Die Trocknung erfolgt bei 50°C im Vakuum.

Beispiel 4

65 mol% Methylmethacrylat, 18 mol% Methacrylsäure, 12 mol% Natriumstyrolsulfonat und 5 mol% Cinnamoylethylmethacrylat werden unter Schutzgas in Dimethylsulfoxid gelöst. Nach Erreichen der Reaktionstemperatur von 75°C werden 0,6 mol% Azobisisobutyronitril, gelöst in Dimethylsulfoxid, zugetropft. Nach 16 Stunden Reaktionsdauer wird das Produkt mit Eiswasser ausgefällt und anschließend im Soxhlet mit Aceton und Wasser extrahiert. Die Trocknung erfolgt bei 50°C im Vakuum.

Beispiel 5

80 mol% Methylmethacrylat, 10 mol% Acrylsäure, 5 mol% Natriumstyrolsulfonat und 5 mol% Cinnamoylethylmethacrylat werden unter Schutzgas in Dimethylsulfoxid vorgelegt. Nach Erreichen der Reaktionstemperatur von 75°C werden 0,6 mol% Azobissobutyrontril, gelöst in Dimethylsulfoxid, zugetropft. Nach 16 Stunden Reaktionsdauer wird das Produkt mit Eiswasser ausgefällt und anschließend im Soxhlet mit Aceton und Wasser extrahiert. Die Trocknung erfolgt bei 50°C im Vakuum.

Beispiel 6

87,5 mol% Methylmethacrylat, 5 mol% Maleinsäureanhydrid, 2,5 mol% Natriumstyrolsulfonat und 5 mol% Cinnamoylethylmethacrylat werden unter Schutzgas in Dimethylsulfoxid vorgelegt. Nach Erreichen der Reaktionstemperatur von 70°C werden 0,6 mol% Azobisisobutyronitril, gelöst in Dimethylsulfoxid, zugetropft. Nach 16 Stunden Reaktionsdauer wird das Produkt mit Eiswasser ausgefällt und anschließend im Soxhlet mit Aceton und Wasser extrahiert. Die Trocknung erfolgt bei 50°C im Vakuum.

Beispiel 7

80 mol% Methylmethacrylat, 8 mol% Methacrylsäure, 7 mol% Natriumstyrolsulfonat und 5 mol% Cinnamoylethylmethacrylat werden unter Schutzgas in Dimethylsulfoxid vorgelegt. Nach Erreichen der Reaktionstemperatur von 70°C werden 0,6 mol% Azobisisobutyronitril, gelöst in Dimethylsulfoxid, zugetropft. Nach 16 Stunden Reaktionsdauer wird das Produkt mit Eiswasser ausgefällt und anschließend im Soxhlet mit Aceton und Wasser extrahiert. Die Trocknung erfolgt bei 50°C im Vakuum.

Beispiel 8

85 mol% Methylmethacrylat, 7,5 mol% Maleinsäureanhydrid, 2,5 mol% Natriumstyrolsulfonat und 5 mol% Cinnamoylethylmethacrylat werden unter Schutzgas in Dimethylsulfoxid vorgelegt. Nach Erreichen der Reaktionstemperatur von 70°C werden 0,6 mol% Azobisisobutyronitril, gelöst in Dimethylsulfoxid, zugetropft. Nach 18 Stunden Reaktionsdauer wird das Produkt mit Eiswasser ausgefällt und anschließend im Soxhlet mit Aceton und Wasser extrahiert. Die Trocknung erfolgt bei 50°C im Vakuum.

Beispiel 9

65 mol% Methylmethacrylat, 18 mol% Methacrylsäure, 12 mol% Triethylammoniumstyrolsulfonat und 5 mol% Cinnamoylethylmethacrylat werden unter Schutzgas in Dimethylsulfoxid vorgelegt. Nach Erreichen der Reaktionstemperatur von 70°C werden 0.6 mol% Azobisisobutyronitril, gelöst in Dimethylsulfoxid, zugetropft. Nach 16 Stunden Reaktionsdauer wird das Produkt mit Eiswasser ausgefällt und anschließend im Soxhlet mit Aceton und Wasser extrahiert. Die Trocknung erfolgt bei 50°C im Vakuum.

Das als strahlungssensitives Monomer verwendete Cinnamoylethylmethacrylat wird ausgehend von 2-Hydroxyethylmethacrylat (3,8 mM) und Cinnamoylchlorid (3,8 mM) in 100 ml trockenem Ethylether bei Raumtemperatur in Anwesenheit von 3,8 mM Pyridin erhalten.

Beispiel 10

Ein Monomerengemisch aus 59 Mol% Tris(trimethylsiloxy)methacryloyloxypropylsilan (TTMPS), 16 mol% Cinnamoylethylmethacrylat (CEM), 13,7 mol% Methacrylsäure (MA) und 11,3 mol% Dimethyloctylammoniumstyrolsulfonat (DOASS) wird in THF als Lösemittel unter Stickstoff als Schutzgas vorgelegt und auf 65°C erhitzt. Nach Erreichen dieser Temperatur werden 0,6 mol% Azobisisobutyronitril (AIBN) in THF als Lösemittel über einen Zeitraum von einer Stunde zudosiert. Nach einer Reaktionsdauer von 24 Stunden wird das Quaterpolymer durch Entfernen des Lösemittels am Rotationsverdampfer isoliert und anschließend mit Wasser gewaschen. Die NMR-Analyse des Produkts ergibt eine Zusammensetzung von

| TTMPS | CEM | MA | DOASS | |
|---|---|---|---|---|
| 66,7 | 13,1 | 11,6 | 8,6 | mol% |

Das Verhältnis von COOH bzw. $COO^-$ zu $SO_3^-$ beträgt 1,4.

Beispiel 11

Ein Monomerengemisch aus 60,4 Mol% Tris(trimethylsiloxy)methacryloyloxypropylsilan (TTMPS), 18 mol% Cinnamoylethylmethacryiat (CEM), 9,5 mol% Methacrylsäure (MA) und 12,1 mol% Dimethyloctyiammoniumstyrolsulfonat (DOASS) wird in THF als Lösemittel unter Stickstoff als Schutzgas vorgelegt und auf 65°C erhitzt. Nach Erreichen dieser Temperatur werden 0,6 mol% Azobisisobutyronitril (AIBN) in THF als Lösemittel über einen Zeitraum von einer Stunde zudosiert. Nach einer Reaktionsdauer von 24 Stunden wird das Quaterpolymer durch Entfernen des Lösemittels am Rotationsverdampfer isoliert und anschließend mit Wasser gewaschen. Die NMR-Analyse des Produkts ergibt eine Zusammensetzung von

| TTMPS | CEM | MA | DOASS | |
|---|---|---|---|---|
| 68,5 | 13,8 | 7,6 | 10,1 | mol% |

Das Verhältnis von COOH bzw. $COO^-$ zu $SO_3^-$ beträgt 0,8.

(4) Pfropfung der Beschichtungspolymeren auf Substratfolien

Zur Pfropfung werden die zuvor hergestellten cinnamoylgruppenhaltigen Beschichtungspolymeren eingesetzt. Die

Beschichtung der Substrate wurde mittels Photografting durchgeführt. Die Beschichtungspolymeren der Beispiele 1 bis 9 wurden auf aktivierte, die Beschichtungspolymeren der Beispiele 10 und 11 auf nicht aktivierte polymere Substrate gepropft.

Bei der Pfropfung der Beschichtungspolymeren auf aktivierte Oberflächen wurde wie folgt verfahren:

- Die Aktivierung wird durch UV-Bestrahlung mit einer Hg-Dampflampe (100W) durchgeführt, die Pfropfung wird durch Bestrahlung mit derselben Lampe initiiert.
- Die verschiedenen Substratfolien werden 20 min bestrahlt und anschließend 15 min in THF getaucht.
- Eine Lösung des Beschichtungspolymers (10 g/l) in THF/Dimethylsulfoxid (80/20) wird auf 2 Proben der Substratfolie aufgesprüht.
- Die beiden Proben werden 10 min bestrahlt.

Die Pfropfung auf nicht aktivierte Oberflächen wurde wie folgt durchgeführt:

- 2 Proben einer Substratfolie aus Silicon (Polysiloxan) werden mit einer Lösung des Quaterpolymers (10 g/l) in THF/Dimethylsulfoxid (80:20) besprüht.
- Die Proben werden 10 min mit einer Quecksilberdampflampe von 100 W bestrahlt (Abstand der Probe zur Lampe 2 cm).
- Die Pfropfung wird dadurch nachgewiesen, daß das Substrat nach Extraktion mit Wasser (6 h bei 60°C) eine Gewichtszunahme um 15,9% (Beispiel 10) bzw. 18,3% (Beispiel 11) aufweist.

Es kommt zu einer Vernetzung und Pfropfung mittels der α-ständigen Doppelbindungen:

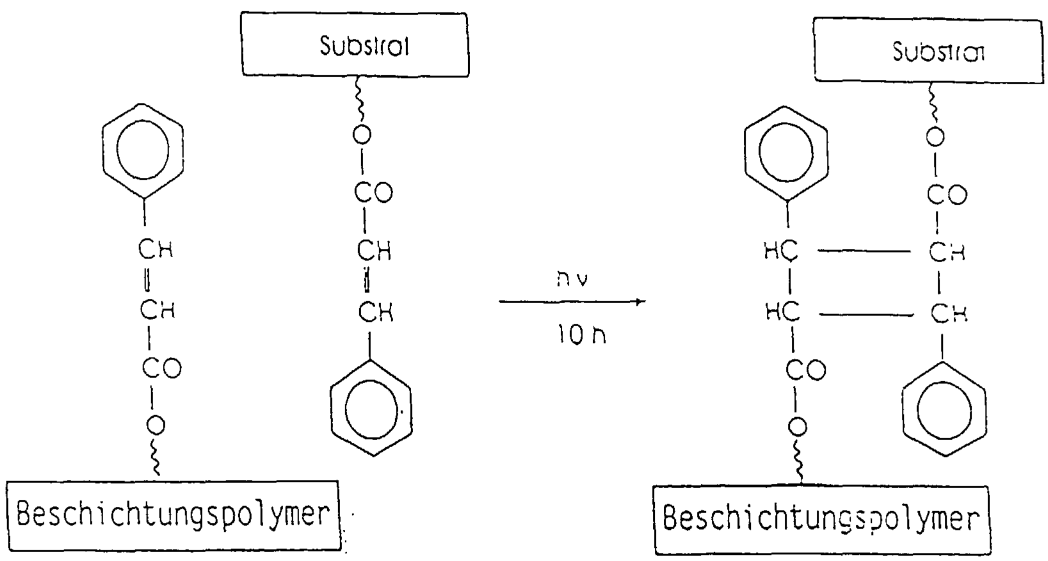


Die Photovernetzung mittels der strahlungssensitiven Gruppen kann IR-spektroskopisch beobachtet werden. Während das IR-Spektrum des bereits mit dem Beschichtungspolymer beschichteten, aber noch nicht UV-bestrahlten Substrats bei 1637 $cm^{-1}$ eine den C=C-Doppelbindungen zugeordnete Bande aufweist, ist diese Bande nach der UV-Bestrahlung nicht mehr registrierbar.

(5) Ergebnisse der Prüfung auf bioaktive Eigenschaften

Die Ergebnisse der Prüfung auf bioaktive Eigenschaften der beschichteten Substratfolien sind aus den folgenden 7 graphischen Darstellungen ersichtlich. Die Figuren 1 bis 6 demonstrieren die bakterienabweisenden Eigenschaften der erfindungsgemäßen Beschichtungspolymeren. Die Szintillationswerte wurden mit (nicht erfindungsgemäßen) Folien aus Beschichtungspolymeren erhalten. Man erkennt, daß die bakterienabweisenden Eigenschaften der erfindungsgemäß beschichteten Substratfolien denjenigen der Folien aus den Beschichtungspolymeren sehr ähnlich sind.

Aus der Figur 7 ist ersichtlich, daß im Bereich des Verhältnisses $CO_2^-/SO_3^-$ bis zu etwa 3 eine deutliche Reduktion des Zellwachstums eintritt. während um Bereich zwischen etwa 2 und etwa 5 die Zellproliferation etwa derjenigen der

unbeschichteten Folie entspricht und jedenfalls erheblich weniger vermindert ist als die Bakterienadhäsion in demselben Bereich.

Figur 1

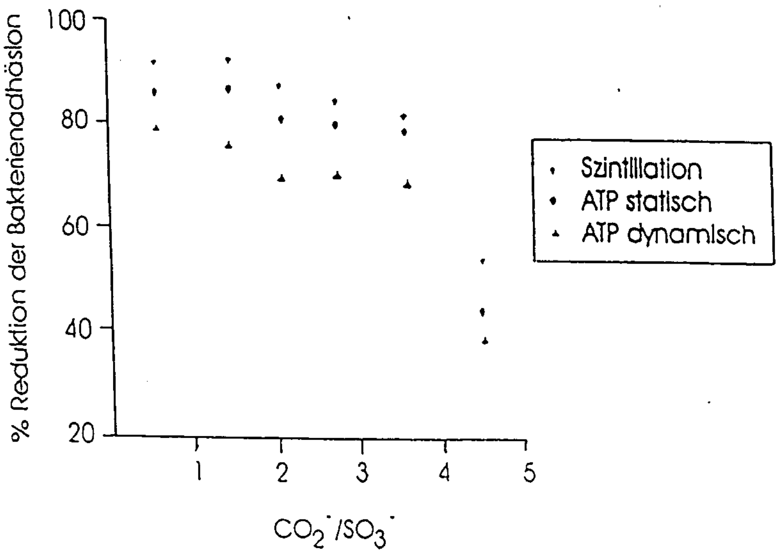


Reduktion der Adhäsion von Staphylococcus aureus auf erfindungsgemäß beschichteten Folien in Abhängigkeit von dem molaren $COO^-/SO_3^-$-Verhältnis

Figur 2

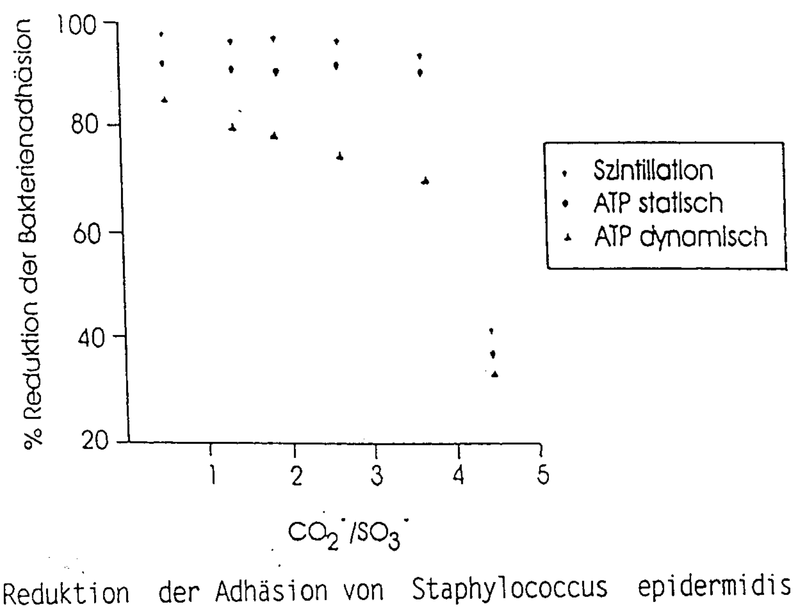


Reduktion der Adhäsion von Staphylococcus epidermidis auf erfindungsgemäß beschichteten Folien in Abhängigkeit von dem molaren $COO^-/SO_3^-$-Verhältnis

Figur 3

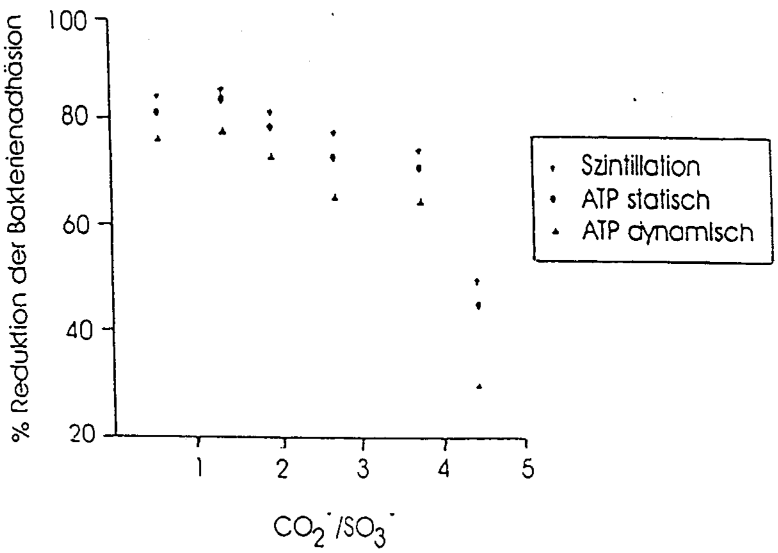


Reduktion der Adhäsion von Staphylococcus pyogenes auf erfindungsgemäß beschichteten Folien in Abhängigkeit von dem molaren $COO^-/SO_3^-$-Verhältnis

Figur 4

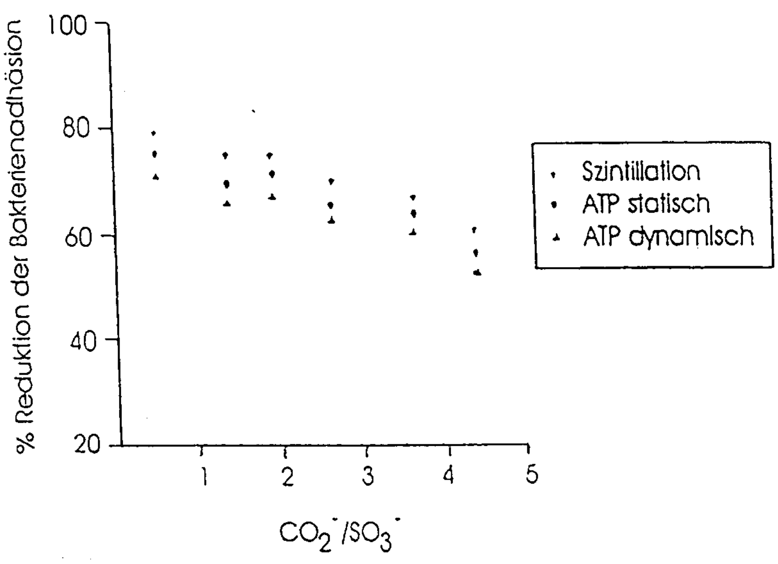


Reduktion der Adhäsion von Klebsiella pneumoniae auf erfindungsgemäß beschichteten Folien in Abhängigkeit von dem molaren $COO^-/SO_3^-$-Verhältnis

Figur 5

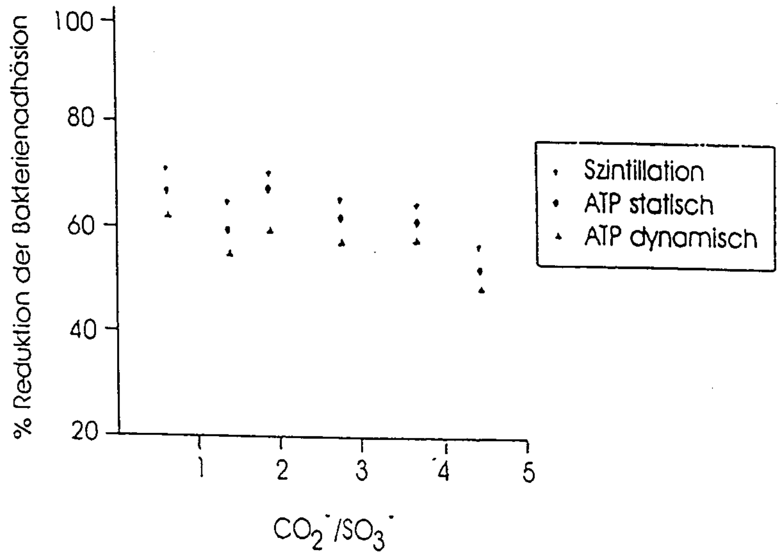


Reduktion der Adhäsion von Pseudomonas aeroginosa auf erfindungsgemäß beschichteten Folien in Abhängigkeit von dem molaren $COO^-/SO_3^-$-Verhältnis

Figur 6

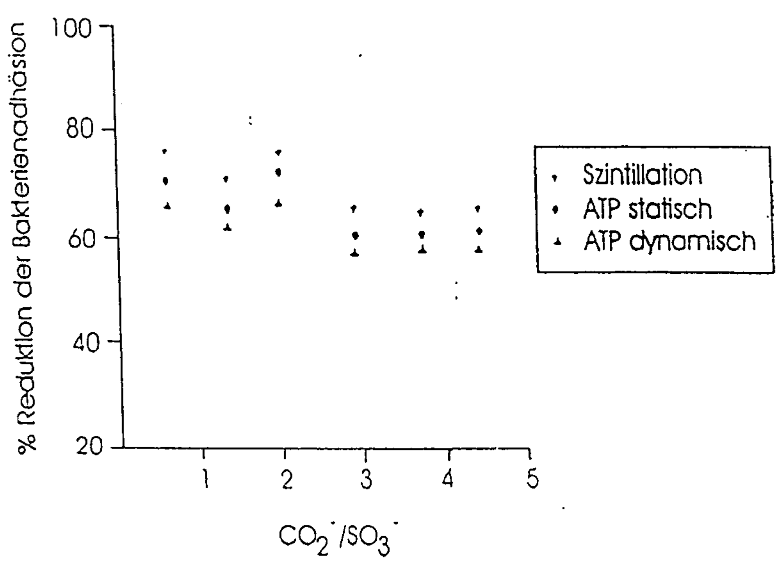


Reduktion der Adhäsion von Escherichia coli auf erfindungsgemäß beschichteten Folien in Abhängigkeit von dem molaren $COO^-/SO_3^-$-Verhältnis

Figur 7

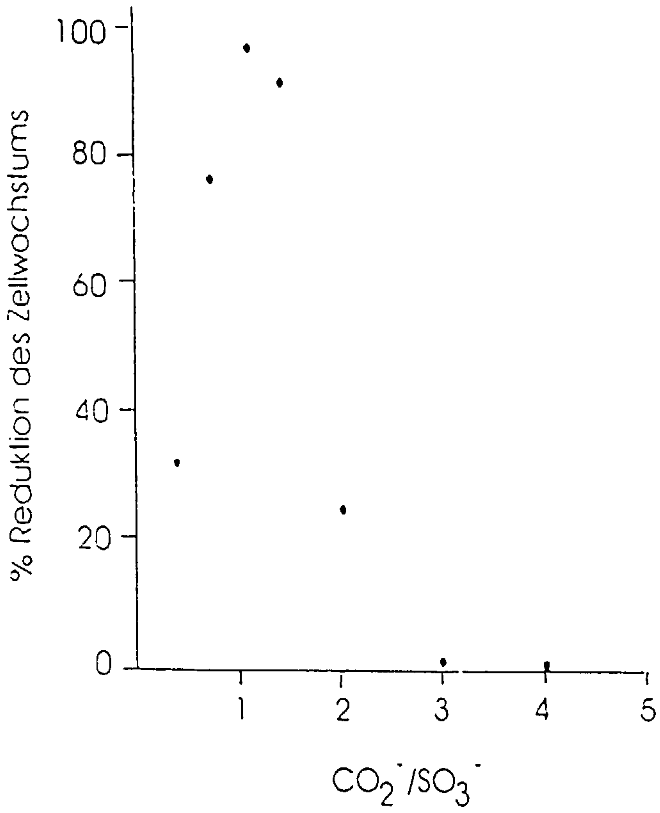


Reduktion des Zellwachstums von menschlichen Fibroblasten auf beschichteten Folien in Abhängigkeit von dem molaren $COO^-/SO_3^-$-Verhältnis

## Patentansprüche

1. Verfahren zur Herstellung von bioaktiven, kovalent fixierten Beschichtungen auf der Oberfläche von Substraten, dadurch gekennzeichnet, daß man ein Beschichtungspolymer, das

   (i) mindestens ein Monomer der allgemeinen Formel $R\text{-}(A)_a$ (I), in der

   R einen ein- oder zweifach olefinisch ungesättigten organischen Rest, vorzugsweise einen Kohlenwasserstoffrest, mit der Wertigkeit a bedeutet, nischen Rest, vorzugsweise einen Kohlenwasserstoffrest, mit der Wertigkeit a bedeutet,
   A eine Carboxylgruppe -COOH, Schwefelsäuregruppe $-OSO_2OH$, Sulfonsäuregruppe $-SO_3H$, Phosphorsäuregruppe $-OPO(OH)_2$, Phosphonsäuregruppe $-PO(OH)_2$, Phosphorigsäuregruppe $-0P(OH)_2$, phenolische Hydroxylgruppe oder ein Salz einer der genannten sauren Gruppen bezeichnet, und
   a für 1, 2 oder 3 steht; und

   (ii) mindestens ein Monomer mit einer UV-strahlungssensitiven Gruppe
   einpolymerisiert enthält, strahleninduziert auf eine gegebenenfalls aktivierte Substratoberfläche pfropft.

**2.** Verfahren nach Anspruch 1, dadurch gekennzeichnet, daß das Metallion ein Alkalimetallion ist.

**3.** Verfahren nach Anspruch 2, dadurch gekennzeichnet, daß das Alkalimetallion ein Natriumion ist.

**4.** Verfahren nach einem der Ansprüche 1 bis 3, dadurch gekennzeichnet, daß das Beschichtungspolymer jeweils mindestens ein Monomer der allgemeinen Formeln II oder III,

$$(C_nH_{2n-q-x})(COOR^1)_x \quad \text{Formel II}$$

$$(C_nH_{2n-q-x})(SO_3R^1)_x \quad \text{Formel III}$$

enthält, in denen

- $\underline{n}$ jeweils unabhängig für eine ganze Zahl von 2 bis einschließlich 6;
- $\underline{x}$ jeweils unabhängig für 1 oder 2;
- $\underline{q}$ jeweils unabhängig für 0 oder 2 steht; und
- $R^1$ jeweils unabhängig -H oder ein Äquivalent eines Metallions bedeutet.

**5.** Verfahren nach einem der Ansprüche 1 bis 3, dadurch gekennzeichnet, daß das Beschichtungspolymer mindestens ein von Benzol abgeleitetes Monomer der allgemeinen Formel IV

$$(C_6H_{6-b-c-d})B_bR^3{}_c(OH)_d \quad \text{Formel IV}$$

enthält, worin

- B jeweils unabhängig einen ein- oder zweifach olefinisch ungesättigten geradkettigen oder verzweigten Rest der Formeln $-(C_nH_{2n-1-q-x})(COOR^1)_x$ oder $-(C_nH_{2n-1-q-x})(SO_3R^1)_x$ bedeutet, wobei $R^1$, $\underline{n}$, $\underline{q}$ und $\underline{x}$ wie in Anspruch 1 definiert sind;
- $R^3$ jeweils unabhängig $C_{1-4}$-Alkyl, $-NH_2$, -COOH, $-SO_3H$, $-OSO_3H$, $-OPO(OH)_2$, $-PO(OH)_2$, $-OP(OH)_2$, $-OPO(O^-)OCH_2CH_2N^-(CH_3)_3$, $-PO(O^-)OCH_2CH_2N^+(CH_3)_3$, $-OP(O^-)OCH_2CH_2N^+(CH_3)_3$ oder ein Salz einer der genannten Gruppen bedeutet;
- $\underline{b}$ für 1, 2, oder 3 steht;
- $\underline{c}$ für 0, 1, 2, oder 3 steht; und
- $\underline{d}$ für 0, 1, 2, oder 3 steht;
  mit der Maßgabe, daß $\bar{b} + \bar{c} + \bar{d} \leq 6$, vorteilhaft $\leq 4$ ist.

**6.** Verfahren nach einem der Ansprüche 1 bis 3, dadurch gekennzeichnet, daß das Beschichtungspolymer mindestens ein Monomer enthält, das aus der Gruppe ausgewählt ist, die aus der Formel I entsprechenden olefinisch ungesättigten sauren Schwefelsäureestern und deren Salzen; Sulfonsäuren und deren Salzen; Phosphonsäuen und deren neutralen oder sauren Salzen; Phosphorsäureestern und deren neutralen oder sauren Salzen; und Phosphorigsäuren und deren neutralen oder sauren Salzen besteht.

**7.** Verfahren nach einem der Ansprüche 1 bis 6, dadurch gekennzeichnet, daß wenn das Beschichtungspolymer ein Monomer I bis IV mit einer Carboxylgruppe oder einer Carboxylatgruppe enthält, dieses Monomer mindestens einen weiteren Rest A mit einer anderen der für A genannten Bedeutungen aufweist oder das Beschichtungspolymer mindestens ein weiteres Monomer I bis IV enthält, in dem A eine andere der für A genannten Bedeutungen hat.

**8.** Verfahren nach Anspruch 7, dadurch gekennzeichnet, daß das Beschichtungspolymer (a) ein Monomer mit Carbonsäure- und/oder Carboxylatgruppe und (b) ein Monomer mit Sulfonsäure und/oder Sulfonatgruppen enthält, wobei die molaren Anteile dieser Monomeren zusammen 5 bis 40 % betragen.

**9.** Verfahren nach Anspruch 7, dadurch gekennzeichnet, daß das Molverhältnis der Monomeren (a) zu den Monomeren (b) <10 ist.

**10.** Verfahren nach Anspruch 7, dadurch gekennzeichnet, daß das Molverhältnis der Monomeren (a) zu den Monomeren (b) 0,5 bis 10 beträgt.

11. Verfahren nach Anspruch 7, dadurch gekennzeichnet, daß das Molverhältnis der Monomeren (a) zu den Monomeren (b) 0,4 bis 3 beträgt und das Beschichtungspolymer bakterienabweisend und zugleich zellproliferationsinhibierend ist.

12. Verfahren nach Anspruch 7, dadurch gekennzeichnet, daß das Molverhältnis der Monomeren (a) zu den Monomeren (b) 2 bis 5 beträgt und das Beschichtungspolymer bakterienabweisend und zugleich zellproliferationsfördernd ist.

13. Verfahren nach einem der Ansprüche 1 bis 12, dadurch gekennzeichnet, daß das Monomer mit UV-strahlungssensitiver Gruppe ein Cinnamoyl- oder Furylderivat ist.

14. Verfahren nach Anspruch 13, dadurch gekennzeichnet, daß das Monomer mit UV-strahlungssensitiver Gruppe ein Cinnamoylethylacrylat oder -methacrylat ist.

15. Verfahren nach einem der Ansprüche 1 bis 14, dadurch gekennzeichnet, daß die Substratoberfläche durch ein einpolymerisiertes Monomer mit einer UV-strahlungssensitiven Gruppe aktiviert wird.

16. Verfahren nach einem der Ansprüche 1 bis 14, dadurch gekennzeichnet, daß die Substratoberfläche durch UV-Strahlung aktiviert wird.